# EUROPEAN PATENT APPLICATION

(11) **EP 4 473 986 A1**
(43) Date of publication of application: **11.12.2024**
(21) Application number: 23752875.7
(22) Date of filing: 07.02.2023
(51) Int. Cl.: A61M 5/142

(54) **INFUSION PUMP**

(30) Priority: 14.02.2022 JP 2022020838
(71) Applicant: Terumo Kabushiki Kaisha, Tokyo 151-0072 (JP)
(72) Inventor: IDE, Jun, Ashigarakami-gun, Kanagawa 259-0151 (JP); HARADA, Katsunori, Tokyo 106-6135 (JP)
(74) Representative: TBK
(86) International application number: PCT/JP2023/004057
(87) International publication number: WO 2023/153411

(57) **Abstract**

An infusion pump according to the present disclosure includes: a main body unit including a tube attachment unit to which an infusion tube is attached substantially horizontally; and a door unit that is openable and closable with respect to the main body unit and covers the tube attachment unit in a state of being closed with respect to the main body unit, in which: the tube attachment unit includes an indwelling portion configured to place the infusion tube on an upper side in a height direction of the main body unit, and a first protruding portion that lies in a row with the indwelling portion, extends from the indwelling portion toward a lower side in the height direction of the main body unit, and includes a slope in which a degree of protrusion from the main body unit is larger at a position closer to the lower side in the height direction; the door unit includes a second protruding portion that protrudes toward the main body unit in a state where the door unit is closed with respect to the main body unit; and the second protruding portion is configured to lift the infusion tube to the upper side in the height direction along the slope of the first protruding portion and place the infusion tube on the indwelling portion when the door unit is closed with respect to the main body unit.

## Description

### Technical Field

The present disclosure relates to an infusion pump.

### Background Art

Conventionally, a technique is known for improving ease of attachment of an infusion tube to an infusion pump, in the infusion pump for delivering a drug or the like to a patient. For example, in Patent Literature 1, an infusion pump is disclosed that includes an infusion tube guide part for fitting an infusion tube when the infusion tube is attached to the infusion pump.

### Citation List

### Patent Literature

Patent Literature 1: JP 2012-196411 A

### Summary of Invention

### Technical Problem

However, in the infusion pump disclosed in Patent Literature 1, it is necessary for users to confirm that the infusion tube is fitted into the infusion tube guide part visually or in other ways. For that reason, further improvement is required of the ease of attachment of the infusion tube to the infusion pump.

An object of the present disclosure made in view of such circumstances is to provide an infusion pump that improves ease of attachment of an infusion tube on the infusion pump.

### Solution to Problem

An infusion pump according to an embodiment of the present disclosure includes: a main body unit including a tube attachment unit to which an infusion tube is attached substantially horizontally; and a door unit that is openable and closable with respect to the main body unit and covers the tube attachment unit in a state of being closed with respect to the main body unit, in which: the tube attachment unit includes an indwelling portion configured to place the infusion tube on an upper side in a height direction of the main body unit, and a first protruding portion that lies in a row with the indwelling portion, extends from the indwelling portion toward a lower side in the height direction of the main body unit, and includes a slope in which a degree of protrusion from the main body unit is larger at a position closer to the lower side in the height direction; the door unit includes a second protruding portion that protrudes toward the main body unit in a state where the door unit is closed with respect to the main body unit; and the second protruding portion is configured to lift the infusion tube to the upper side in the height direction along the slope of the first protruding portion and place the infusion tube on the indwelling portion when the door unit is closed with respect to the main body unit.

In the infusion pump according to the embodiment of the present disclosure, it is preferable that the slope of the first protruding portion is curved in an arc shape such that the degree of protrusion from the main body unit is larger at a position closer to the lower side in the height direction.

In the infusion pump according to the embodiment of the present disclosure, it is preferable that the indwelling portion is provided at a position that is on a side surface in a width direction of the main body unit and is not covered by the door unit, in a state where the door unit is closed with respect to the main body unit.

In the infusion pump according to the embodiment of the present disclosure, it is preferable that the tube attachment unit includes a tube clamp part configured to clamp a part of the infusion tube to fix the infusion tube to the tube attachment unit, and the first protruding portion is provided at a position where the first protruding portion is enabled to come into contact with the infusion tube in a case where the infusion tube fixed by the tube clamp part is in a state of hanging down to the lower side in the height direction by an own weight of the infusion tube.

In the infusion pump according to the embodiment of the present disclosure, it is preferable that the tube clamp part is configured to hold the infusion tube in an occlusion state in a state where the door unit is opened with respect to the main body unit, and hold the infusion tube in an occlusion release state in a state where the door unit is closed with respect to the main body unit.

In the infusion pump according to the embodiment of the present disclosure, it is preferable that the tube clamp part is detachable from the tube attachment unit.

In the infusion pump according to the embodiment of the present disclosure, it is preferable that the second protruding portion is located on an opposite side from the tube clamp part with the first protruding portion interposed therebetween in the width direction of the main body unit in a state where the door unit is closed with respect to the main body unit.

### Advantageous Effects of Invention

According to the present disclosure, it is possible to provide an infusion pump that improves ease of attachment of an infusion tube to the infusion pump.

### Brief Description of Drawings

Fig. 1 is a diagram illustrating an infusion line including an infusion pump as an embodiment of the present disclosure.
Fig. 2 is a perspective view of the infusion pump illustrated in Fig. 1.
Fig. 3 is a front view of a state where a door unit of the infusion pump illustrated in Fig. 1 is closed.
Fig. 4 is a front view of a state where the door unit of the infusion pump illustrated in Fig. 1 is opened.
Fig. 5 is a side view of the infusion pump illustrated in Fig. 4.
Fig. 6 is an enlarged perspective view of a downstream-side tube guide part and the vicinity thereof of the infusion pump illustrated in Fig. 4.
Fig. 7 is a side view of the infusion pump illustrated in Fig. 3.
Fig. 8 is a block diagram of the infusion pump illustrated in Fig. 1.
Fig. 9 is a diagram illustrating a tube clamp part of the infusion pump illustrated in Fig. 4.
Fig. 10 is a cross-sectional view of the infusion pump taken along a line I-I' illustrated in Fig. 4.

### Description of Embodiments

Hereinafter, an infusion pump according to an embodiment of the present disclosure will be described with reference to the drawings. In the drawings, common members and parts are denoted by the same reference signs. In the description of the present embodiment, the description of the common members and parts will be omitted or simplified as appropriate.

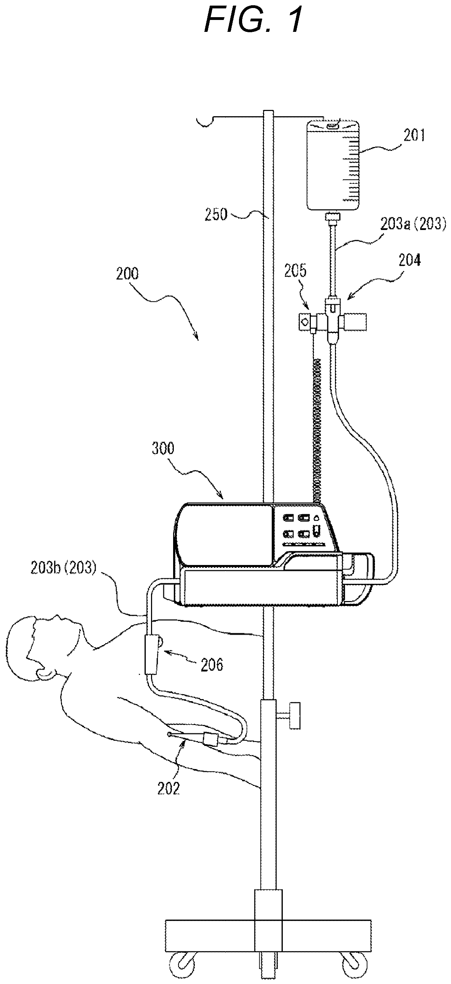

Fig. 1 is a diagram illustrating an infusion line 200 formed by connecting an infusion container 201 containing a liquid such as a drug solution to an indwelling needle 202 indwelled in a state of puncturing a patient with a tube 203 as an infusion tube. Hereinafter, the infusion container 201 side of the infusion line 200 may be referred to as a "flow path upstream side". In addition, the indwelling needle 202 side of the infusion line 200 may be referred to as a "flow path downstream side". Further, hereinafter, a direction from the flow path upstream side to the flow path downstream side of the infusion line 200 may be referred to as a liquid delivery direction A.

More specifically, the infusion line 200 illustrated in Fig. 1 includes an infusion container 201 hung on a stand 250, a drip tube 204, an indwelling needle 202, and two tubes 203. The two tubes 203 of the infusion line 200 are a first tube 203a connecting the infusion container 201 to the drip tube 204, and a second tube 203b connecting the drip tube 204 to the indwelling needle 202. In Fig. 1, a drip probe as a number-of-drops abnormality detection apparatus 205 is attached to the drip tube 204 of the infusion line 200. In addition, in Fig. 1, an infusion pump 300 as a liquid delivery pump is attached to the second tube 203b of the infusion line 200. In addition, the infusion pump 300 is fixed to the stand 250. Further, a clamp 206 is attached to the second tube 203b of the infusion line 200 illustrated in Fig. 1 on the flow path downstream side from the infusion pump 300. In the following description, in a case where the first tube 203a and the second tube 203b are not particularly distinguished from each other, the tubes are simply collectively referred to as the tubes 203.

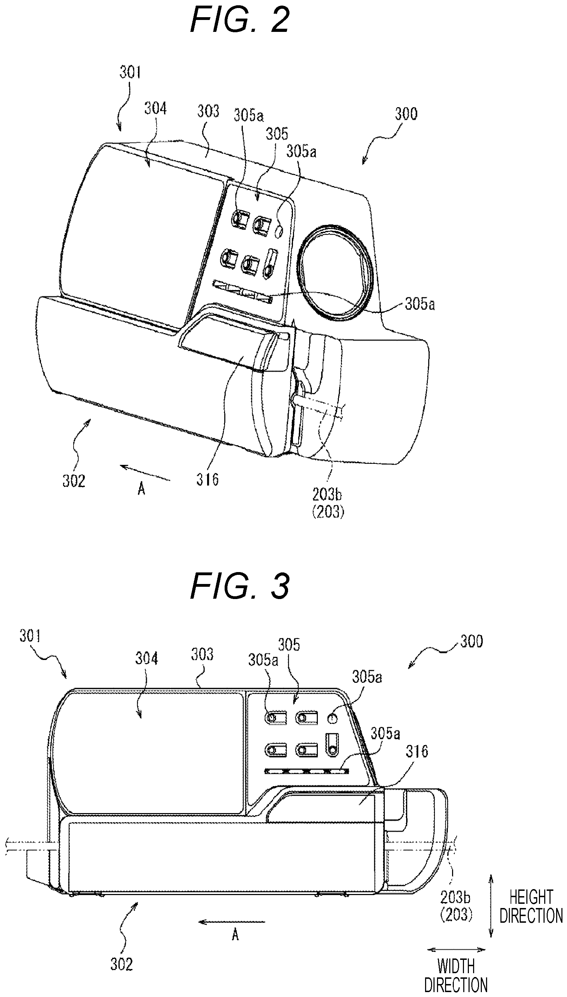

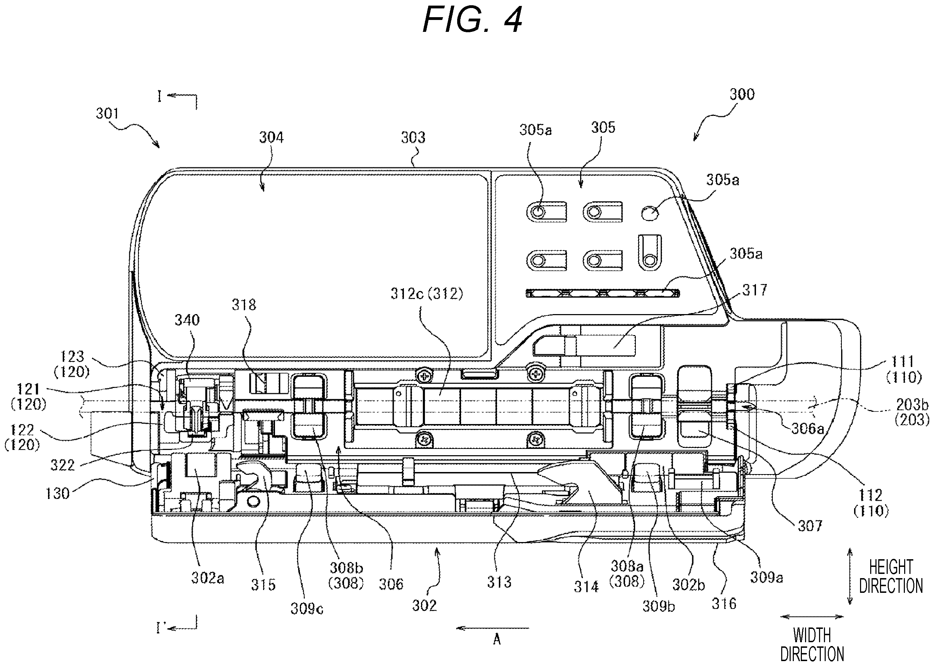

First, a schematic configuration will be described of the infusion pump 300 with reference to Figs. 2, 3, and 4. Fig. 2 is a perspective view of the infusion pump 300. Fig. 3 is a front view of a state where a door unit 302 of the infusion pump 300 is closed with respect to a main body unit 301. Fig. 4 is a front view of a state where the door unit 302 of the infusion pump 300 is opened with respect to the main body unit 301. In Figs. 2 to 4, the second tube 203b of Fig. 1 held by the infusion pump 300 is indicated by a two-dot chain line for convenience of description. In Figs. 3 and 4, the infusion pump 300 is illustrated in a front view of the infusion pump 300. In Figs. 3 and 4 (front view of the infusion pump 300), the left-right direction is also referred to as a "width direction" of the infusion pump 300, and the up-down direction is also referred to as a "height direction" of the infusion pump 300. The front-back direction is also referred to as a "depth direction" of the infusion pump 300.

The infusion pump 300 illustrated in Figs. 2 to 4 is a liquid delivery pump used, for example, in an intensive care unit (ICU, CCU, NICU) or the like, and used to inject a liquid such as an anticancer agent, an anesthetic, a chemotherapeutic agent, a blood transfusion, or a nutrient for a patient for a relatively long time with an accuracy within ±10% (preferably an accuracy within ±3%) in a range of about 1 to 1200 mL/h (amount of injection per hour, that is, injection rate), 1 to 9999 mL (scheduled amount of injection).

The infusion pump 300 includes the main body unit 301 and the door unit 302 that can be opened and closed with respect to the main body unit 301. A main body cover 303 that is an exterior cover of the main body unit 301, and the door unit 302 described above are molded using a molding resin material having chemical resistance.

First, elements will be described arranged in the main body cover 303 of the main body unit 301 of the infusion pump 300. As illustrated in Figs. 2 to 4, a display unit 304 and an operation panel unit 305 are arranged on a front portion of the main body cover 303. The display unit 304 is an image display apparatus such as a color liquid crystal display apparatus. The display unit 304 is located on the left side of a front upper portion of the main body cover 303 and is arranged above the door unit 302.

On the display unit 304, a scheduled amount of injection (mL) of administration, an integrated amount (mL) of administration, a charge history, an injection rate (mL/h), and the like are displayed. In addition, a warning message may be displayed on the display unit 304.

The operation panel unit 305 is arranged on the right side of the front upper portion of the main body cover 303. An operation button 305a is arranged on the operation panel unit 305. The operation button 305a includes, for example, a power switch button, a fast delivery switch button, a start switch button, a stop switch button, and a menu selection button.

As illustrated in Figs. 2 to 4, the door unit 302 is attached to a front lower portion of the main body cover 303 so as to be rotatable about rotation axes of two hinges 302a and 302b. The door unit 302 is a plate-like lid extending long along the left-right direction in the front view of the infusion pump 300 in Figs. 3 and 4. The door unit 302 is configured to be openable and closable with respect to the main body unit 301, and cover a tube attachment unit 306 provided in the front lower portion of the main body cover 303 in a state of being closed with respect to the main body unit 301. As a result, the infusion pump 300 can sandwich and hold the second tube 203b made of a flexible thermoplastic resin such as soft vinyl chloride, for example, between the tube attachment unit 306 and the door unit 302 in a state where the door unit 302 is closed with respect to the main body unit 301 (see Figs. 2 and 3).

The main body unit 301 includes the tube attachment unit 306 to which the infusion tube 203 (the second tube 203b in the present embodiment) is attached substantially horizontally. As illustrated in Fig. 4, the tube attachment unit 306 is provided along the left-right direction below the display unit 304 and the operation panel unit 305 in the front view of the infusion pump 300.

In the present disclosure, "substantially horizontal" preferably means horizontal, but may include being inclined within a range of a predetermined angle with respect to a horizontal direction (a direction perpendicular to a direction in which gravity acts). The range of the predetermined angle is, for example, a range between -10 degrees and 10 degrees, but is not limited thereto.

As illustrated in Fig. 4, the tube attachment unit 306 defines a guide groove 306a to which the second tube 203b is attached. In the present embodiment, the guide groove 306a is continuously provided in the tube attachment unit 306 so as to extend in the left-right direction in the front view of the infusion pump 300, but may be intermittently provided. The second tube 203b is attached to the tube attachment unit 306 along the guide groove 306a. The tube attachment unit 306 includes an air bubble detection sensor 307, an upstream-side occlusion detection sensor 308a, a downstream-side occlusion detection sensor 308b, an upstream-side tube guide part 110, a downstream-side tube guide part 120, a liquid delivery drive part 312, and a tube clamp part 322. More specifically, in the tube attachment unit 306, the upstream-side tube guide part 110, the air bubble detection sensor 307, the upstream-side occlusion detection sensor 308a, the liquid delivery drive part 312, the downstream-side occlusion detection sensor 308b, the tube clamp part 322, and the downstream-side tube guide part 120 are arranged in this order from the upstream side in the liquid delivery direction A along the guide groove 306a. Inside the door unit 302, a tube pressing member 313 and engaging members 314 and 315 are provided.

The tube pressing member 313 is arranged as a long rectangular planar protruding portion along the left-right direction of the infusion pump 300 in the front view (see Fig. 4 and the like), and is at a position facing the liquid delivery drive part 312. The engaging members 314 and 315 are respectively fitted into fitting portions 317 and 318 formed in the main body unit 301 by operation of a lever 316 interlocked with the engaging members 314 and 315. As a result, the door unit 302 can be closed and the tube attachment unit 306 of the main body unit 301 can be closed.

The tube clamp part 322 is configured to clamp a part of the second tube 203b to fix the infusion tube to the tube attachment unit 306.

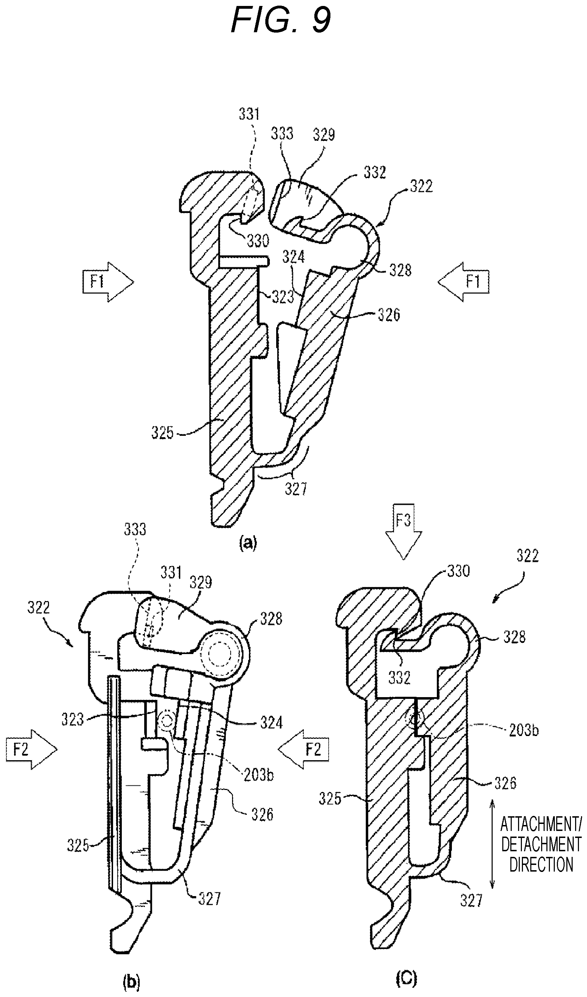

A configuration of the tube clamp part 322 will be described in detail with reference to Fig. 9. Fig. 9 is a diagram illustrating the tube clamp part 322 of the infusion pump 300 illustrated in Fig. 4. Fig. 9(a) is a cross-sectional view of the tube clamp part 322 taken along a line I-I' of Fig. 4. Fig. 9(b) is a plan view of the tube clamp part 322 in a state where occlusion of the second tube 203b is released. Fig. 9(c) is a cross-sectional view of the tube clamp part 322 taken along the line I-I' of Fig. 4 in a state where the second tube 203b is occluded. Hereinafter, a state where occlusion of the second tube 203b is released is also referred to as an occlusion release state of the second tube 203b, and a state where the second tube 203b is occluded is also referred to as an occlusion state of the second tube 203b.

The tube clamp part 322 is detachable from the tube attachment unit 306. Specifically, as illustrated in Fig. 9(c), the tube clamp part 322 is fixed to the tube attachment unit 306 by being inserted into the tube attachment unit 306 along an attachment/detachment direction illustrated in Fig. 9(c) in a state where a part of the second tube 203b is sandwiched between a pair of occlusion portions 323 and 324. In addition, the tube clamp part 322 is detachable from the tube attachment unit 306 by being pulled out along the attachment/detachment direction from the tube attachment unit 306 in a state where a part of the second tube 203b is sandwiched between the pair of occlusion portions 323 and 324. As a result, the tube clamp part 322 can be attached to or detached from the tube attachment unit 306 in a state where the tube clamp part 322 is attached to the second tube 203b in advance and the second tube 203b is occluded.

The tube clamp part 322 includes a main base portion 325 in which one occlusion portion 323 is formed and a sub-base portion 326 in which the other occlusion portion 324 is formed, of the pair of occlusion portions 323 and 324 that sandwiches and occludes a part of the second tube 203b. The main base portion 325 and the sub-base portion 326 are integrally formed continuously via a first elastic support portion 327. The first elastic support portion 327 is elastically deformable. Then, in the natural state, the sub-base portion 326 is urged in a direction away from the main base portion 325 via the first elastic support portion 327. A distal end portion 329 is further formed continuously at an end portion of the sub-base portion 326 on an opposite side from a side continuous with the first elastic support portion 327 via a second elastic support portion 328 having an arc shape. The second elastic support portion 328 is elastically deformable. In the natural state, the distal end portion 329 is urged in a direction away from the occlusion portion 324 of the sub-base portion 326 via the second elastic support portion 328. The tube clamp part 322 includes locking portions 330, 331, 332, and 333 at end portions of the main base portion 325 and the distal end portion 329 on the opposite side from the side continuous with the first elastic support portion 327. Specifically, the main base portion 325 includes the first locking portion 330 protruding in the attachment/detachment direction of the tube clamp part 322 and the second locking portion 331 protruding in a direction orthogonal to the attachment/detachment direction. The distal end portion 329 includes the third locking portion 332 protruding in the attachment/detachment direction and the fourth locking portion 333 protruding in the direction orthogonal to the attachment/detachment direction.

As a result, as illustrated in Fig. 9(a), when external force (F1 in the figure) is applied so as to bring the main base portion 325 and the sub-base portion 326 close to each other so as to sandwich a part of the second tube 203b by the occlusion portions 323 and 324, from a state where the main base portion 325 and the distal end portion 329 are separated from each other, the second locking portion 331 and the fourth locking portion 333 are locked as illustrated in Fig. 9(b). In a state where the second locking portion 331 and the fourth locking portion 333 are locked, the tube clamp part 322 can hold the second tube 203b in the occlusion release state without sandwiching the second tube 203b by the occlusion portions 323 and 324. Next, as illustrated in Fig. 9(b), when external force (F2 in the figure) is further applied so as to bring the main base portion 325 and the sub-base portion 326 close to each other, from a state where the second locking portion 331 and the fourth locking portion 333 are locked, the first locking portion 330 and the third locking portion 332 are locked as illustrated in Fig. 9(c). In a state where the first locking portion 330 and the third locking portion 332 are locked, the tube clamp part 322 can sandwich the second tube 203b by the occlusion portions 323 and 324 and hold the second tube 203b in the occlusion state. Further, as illustrated in Fig. 9(c), when external force (F3 in the figure) is applied in the attachment/detachment direction of the distal end portion 329, from a state where the first locking portion 330 and the third locking portion 332 are locked, locking of the first locking portion 330 and the third locking portion 332 is released, and the tube clamp part 322 returns to the state (occlusion release state) where the second locking portion 331 and the fourth locking portion 333 are locked as illustrated in Fig. 9(b). As described above, the external force F2 or F3 is applied while the second tube 203b is held, whereby the tube clamp part 322 can repeat occluding the second tube 203b or releasing occlusion of the second tube 203b.

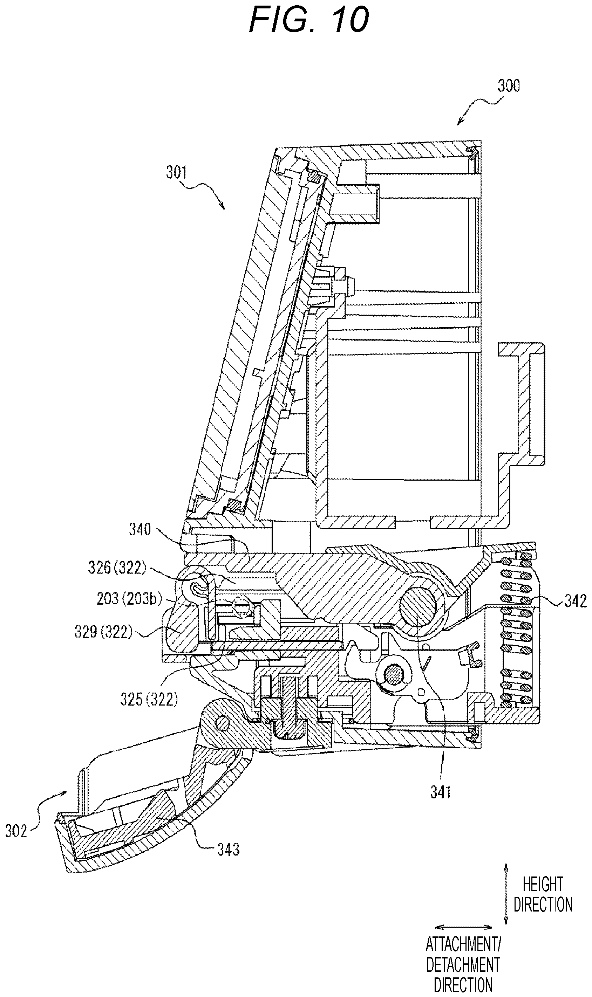

Next, with reference to Fig. 10, a detailed description will be given of deformation of the tube clamp part 322 accompanying opening and closing of the door unit 302 with respect to the main body unit 301. Fig. 10 is a cross-sectional view of the infusion pump 300 taken along the line I-I' illustrated in Fig. 4.

The main body unit 301 includes a rotating arm member 340 having a rod shape extending from the front side to the back side of the main body unit 301. In the natural state, the rotating arm member 340 is urged toward the lower side in the height direction of the main body unit 301 by an urging member 342 provided on the back side of the main body unit 301 about a rotation shaft 341. The urging member 342 is, for example, a spring. In a state where the door unit 302 is opened with respect to the main body unit 301, the tube clamp part 322 is inserted into the tube attachment unit 306 from the front side of the main body unit 301 such that the sub-base portion 326 is in contact with the rotating arm member 340 from the lower side in the height direction of the main body unit 301. As a result, since external force is applied from the rotating arm member 340 to the tube clamp part 322 so that the sub-base portion 326 approaches the main base portion 325, the tube clamp part 322 is in a state where the first locking portion 330 and the third locking portion 332 are locked as illustrated in Fig. 9(c), and can hold the second tube 203b in the occlusion state.

Referring again to Fig. 10, inside the door unit 302, a pressing member 343 is provided configured to come in contact with and press both the rotating arm member 340 and the distal end portion 329 of the tube clamp part 322 in a state where the door unit 302 is closed with respect to the main body unit 301. As a result, when the door unit 302 is closed with respect to the main body unit 301, the pressing member 343 lifts the rotating arm member 340 to the upper side in the height direction of the main body unit 301, and presses the distal end portion 329 toward the back side of the main body unit 301, whereby the locking of the first locking portion 330 and the third locking portion 332 is released and the second locking portion 331 and the fourth locking portion 333 are in a state of being locked in the tube clamp part 322 as illustrated in Fig. 9(b), and the second tube 203b can be held in the occlusion release state.

As described above, the tube clamp part 322 is configured to hold the second tube 203b attached to the tube attachment unit 306 in the occlusion state in a state where the door unit 302 is opened with respect to the main body unit 301, and hold the second tube 203b in the occlusion release state in a state where the door unit 302 is closed with respect to the main body unit 301 As a result, the infusion pump 300 can bring the second tube 203b into the occlusion state at the time of attachment/detachment of the second tube 203b to/from the infusion pump 300, and bring the second tube 203b into the occlusion release state at the time of liquid delivery by the infusion pump 300.

As illustrated in Fig. 4, the upstream-side tube guide part 110 is provided at the right end of the guide groove 306a of the tube attachment unit 306 in the front view of the infusion pump 300, and the downstream-side tube guide part 120 is provided at the left end of the guide groove 306a of the tube attachment unit 306 in the front view of the infusion pump 300. Then, the upstream-side tube guide part 110 can hold the second tube 203b by fitting the flow path upstream side of the second tube 203b of the infusion line 200 (see Fig. 1), and the downstream-side tube guide part 120 can hold the second tube 203b by fitting the flow path downstream side of the second tube 203b of the infusion line 200. That is, the infusion pump 300 can hold the second tube 203b substantially horizontally along the guide groove 306a by the upstream-side tube guide part 110 and the downstream-side tube guide part 120. As described above, the second tube 203b attached substantially horizontally is arranged so as to face the air bubble detection sensor 307, the upstream-side occlusion detection sensor 308a, the liquid delivery drive part 312, the downstream-side occlusion detection sensor 308b, and the tube clamp part 322 in order from the right in the front view of Fig. 4. However, the upstream-side tube guide part 110 and the upstream-side occlusion detection sensor 308a may be arranged at any positions in the guide groove 306a.

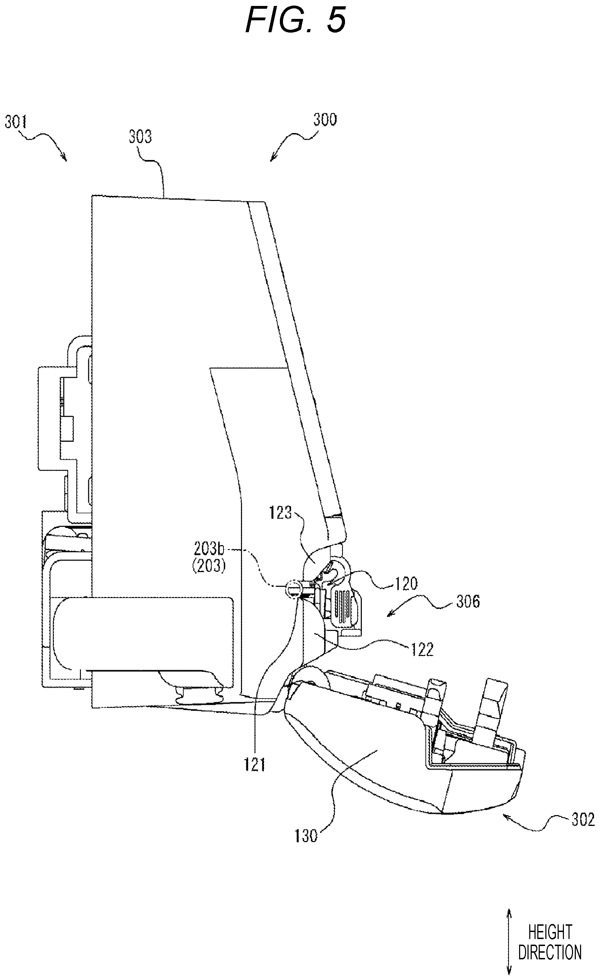

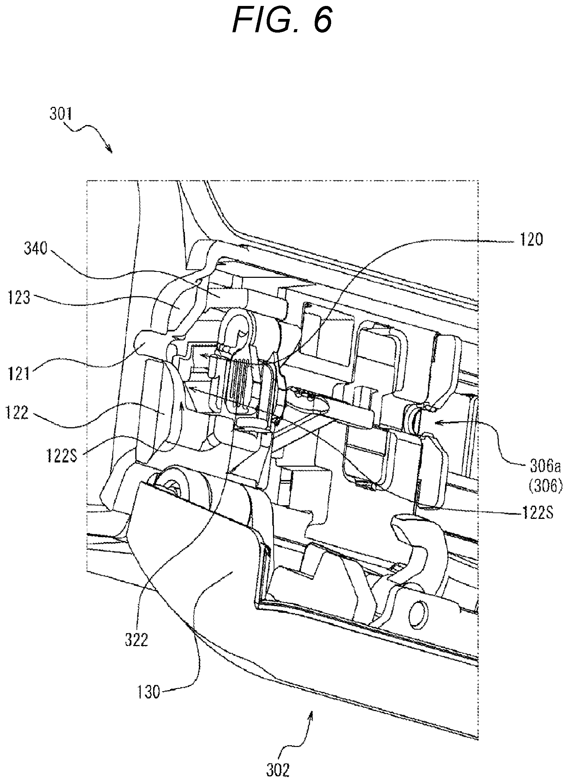

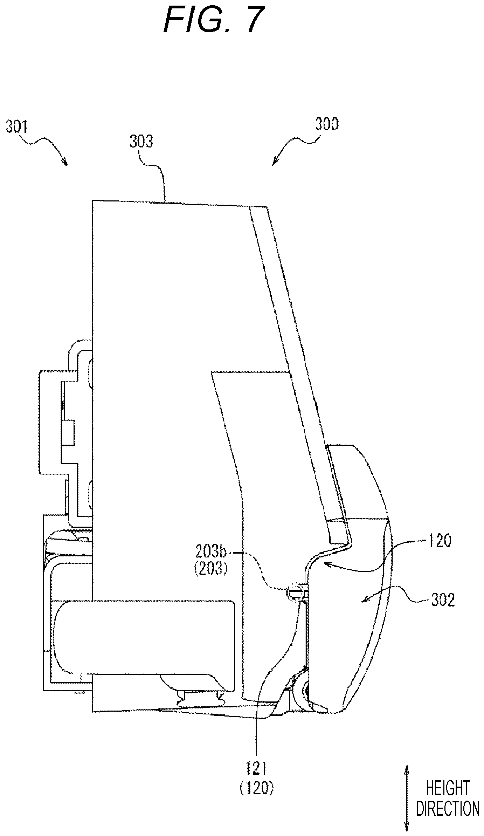

A configuration of the downstream-side tube guide part 120 will be described in detail with reference to Figs. 4, 5, 6, and 7. Fig. 5 is a side view of the infusion pump 300 illustrated in Fig. 4 as viewed from the downstream side in the liquid delivery direction A. Fig. 6 is an enlarged perspective view of the downstream-side tube guide part 120 and the vicinity thereof of the infusion pump 300 illustrated in Fig. 4. Fig. 7 is a side view of the infusion pump 300 illustrated in Fig. 3 as viewed from the downstream side in the liquid delivery direction A.

As illustrated in Figs. 5 and 6, the downstream-side tube guide part 120 includes an indwelling portion 121 and a first protruding portion 122.

The indwelling portion 121 is configured to place the second tube 203b on the upper side in the height direction. The indwelling portion 121 is made to extend in the width direction of the infusion pump 300 so as to form a part of the guide groove 306a, and have a groove shape with an opening on the front side of the infusion pump 300. As a result, the indwelling portion 121 of the downstream-side tube guide part 120 can stably hold the second tube 203b fitted into the downstream-side tube guide part 120.

The first protruding portion 122 is made to have a plate shape, and protrudes from the main body unit 301 to the front side below the indwelling portion 121 in the height direction of the main body unit 301. Specifically, the first protruding portion 122 has a slope 122S that lies in a row with the indwelling portion 121, extends from the indwelling portion 121 toward the lower side in the height direction, and has a larger degree of protrusion from the main body unit 310 at a position closer to the lower side in the height direction of the main body unit 301. As a result, when a user of the infusion pump 300 places the second tube 203b on the indwelling portion 121 of the downstream-side tube guide part 120, the second tube 203b is lifted to the upper side in the height direction along the slope 122S of the first protruding portion 122, whereby the second tube 203b can be attached to the indwelling portion 121. For this reason, the second tube 203b can be easily attached to the infusion pump 300.

More specifically, in the present embodiment, the slope 122S of the first protruding portion 122 is curved in an arc shape such that the degree of protrusion from the main body unit 310 increases at a position closer to the lower side in the height direction of the main body unit 301. As a result, when the user of the infusion pump 300 pushes up the second tube 203b along the slope 122S of the first protruding portion 122 and places the second tube 203b on the indwelling portion 121, the second tube 203b moves mainly in a tangential direction of the first protruding portion 122, so that resistance force such as frictional force is less likely to be applied from the first protruding portion 122 to the second tube 203b. In addition, since the first protruding portion 122 is curved in an arc shape, an inclination of the first protruding portion 122 with respect to the height direction of the main body unit 310 is gentle at a position closer to a portion that lies in a row with the indwelling portion 121, and the second tube 203b placed on the indwelling portion 121 is less likely to be detached from the indwelling portion 121 by its own weight. However, the first protruding portion 122 does not have to be curved in an arc shape.

As illustrated in Fig. 7, the indwelling portion 121 is provided at a position on a side surface in the width direction of the main body unit 301, which is not covered by the door unit 302, in a state where the door unit 302 is closed with respect to the main body unit 301. As a result, it is possible to reduce a risk of occurrence of occlusion of the second tube 203b, underdose of the liquid flowing in the second tube 203b, or the like due to the fact that the second tube 203b is sandwiched between the door unit 302 and the main body unit 301 in a state where the door unit 302 is closed with respect to the main body unit 301.

Referring again to Fig. 6, the first protruding portion 122 is provided at a position where the first protruding portion 122 can come into contact with the second tube 203b in a case where the second tube 203b fixed by the tube clamp part 322 is in a state of hanging down to the lower side in the height direction of the main body unit 301 by its own weight. Specifically, the first protruding portion 122 comes in contact with a portion (a portion adjacent to the downstream side in the present embodiment) adjacent to the upstream side or the downstream side of a part of the second tube 203b clamped by the tube clamp part 322. As a result, after the user of the infusion pump 300 fixes the second tube 203b to the tube attachment unit 306 by the tube clamp part 322, the second tube 203b is easily fitted into the downstream-side tube guide part 120 along the slope 122S of the first protruding portion 122. A distance between the first protruding portion 122 and the tube clamp part 322 in the width direction of the infusion pump 300 may be set, for example, in a range of 9 mm to 15 mm.

The downstream-side tube guide part 120 includes an upper protruding portion 123 in addition to the indwelling portion 121 and the first protruding portion 122. The upper protruding portion 123 is made to have a plate shape, and protrudes from the main body unit 301 toward the front side above the indwelling portion 121 in the height direction of the main body unit 301. Specifically, the upper protruding portion 123 lies in a row with the indwelling portion 121 and extends toward the upper side in the height direction from the indwelling portion 121. The upper protruding portion 123 includes a slope in which the degree of protrusion from the main body unit 310 increases at a position closer to the upper side in the height direction of the main body unit 301. The upper protruding portion 123 faces the first protruding portion 122 in the height direction of the main body unit 301.

The door unit 302 includes a second protruding portion 130 that protrudes toward the main body unit 301 in a state where the door unit 302 is closed with respect to the main body unit 301. In the present embodiment, as illustrated in Figs. 5 and 6, the second protruding portion 130 is made to have a plate shape, and is provided at an end portion on the downstream side in the liquid delivery direction A in the width direction of the door unit 302. The second protruding portion 130 is configured to lift the second tube 203b to the upper side in the height direction of the main body unit 301 along the slope 122S of the first protruding portion 122 and place the second tube 203b on the indwelling portion 121 when the door unit 302 is closed with respect to the main body unit 301. As a result, even in a case where the door unit 302 is closed with respect to the main body unit 301 in a state where the second tube 203b is not placed on the indwelling portion 121, the second protruding portion 130 can lift the second tube 203b along the slope 122S of the first protruding portion 122 and place the second tube 203b on the indwelling portion 121. For this reason, it is possible to reduce the risk of occurrence of occlusion of the second tube 203b, underdose of the liquid flowing in the second tube 203b, or the like due to the fact that the second tube 203b is sandwiched between the door unit 302 and the main body unit 301 when the door unit 302 is closed with respect to the main body unit 301.

As illustrated in Fig. 4, a distance between the first protruding portion 122 and the second protruding portion 130 in the width direction of the infusion pump 300 in a state where the door unit 302 is closed with respect to the main body unit 301 is a distance sufficiently shorter than the diameter of the second tube 203b attached to the infusion pump 300. As a result, when the second protruding portion 130 lifts the first protruding portion 122, the second tube 203b is less likely to be sandwiched between the first protruding portion 122 and the second protruding portion 130. The distance between the first protruding portion 122 and the second protruding portion 130 in the width direction of the infusion pump 300 in a state where the door unit 302 is closed with respect to the main body unit 301 may be set, for example, in a range of 0.3 mm to 1.5 mm.

In addition, as illustrated in Fig. 4, in a state where the door unit 302 is closed with respect to the main body unit 301, the second protruding portion 130 is located on the opposite side from the tube clamp part 322 with the first protruding portion 122 interposed therebetween in the width direction of the main body unit 301. That is, the first protruding portion 122 is located between the second protruding portion 130 and the tube clamp part 322 in the width direction of the main body unit 301 in a state where the door unit 302 is closed with respect to the main body unit 301. As a result, in a case where the second tube 203b is in a state of being fixed by the tube clamp part 322 and hanging down to the lower side in the height direction by its own weight, the second protruding portion 130 more stably lifts the second tube 203b along the slope 122S of the first protruding portion 122. In addition, the second protruding portion 130 constituting the door unit 302 covers the first protruding portion 122 from the outside in the width direction of the main body unit 301, whereby appearance of the infusion pump 300 is improved. However, the second protruding portion 130 may be located between the tube clamp part 322 and the first protruding portion 122 in the width direction of the main body unit 301 in a state where the door unit 302 is closed with respect to the main body unit 301.

As illustrated in Fig. 5, in the first protruding portion 122 of the downstream-side tube guide part 120, a portion where the degree of protrusion from the main body unit 301 increases protrudes to the front side of the main body unit 301 from the rotation axis of the hinges 302a and 302b provided at the front lower portion of the main body unit 301. As a result, when the door unit 302 is closed with respect to the main body unit 301, the second protruding portion 130 rotating about the rotation axes of the hinges 302a and 302b easily lifts the second tube 203b along the slope 122S of the first protruding portion 122.

Referring to Fig. 4, the upstream-side tube guide part 110 includes an upper protruding portion 111 located above the guide groove 306a and a lower protruding portion 112 located below the guide groove 306a in the height direction of the main body unit 301. The upper protruding portion 111 and the lower protruding portion 112 face each other across the guide groove 306a in the height direction of the main body unit 301. As a result, the second tube 203b is fitted between the upper protruding portion 111 and the lower protruding portion 112, whereby the upstream-side tube guide part 110 holds the second tube 203b in the guide groove 306a. In the present embodiment, the upstream-side tube guide part 110 has a configuration different from that of the downstream-side tube guide part 120, but the upstream-side tube guide part 110 may also have a configuration similar to that of the downstream-side tube guide part 120.

The air bubble detection sensor 307 is an apparatus that detects a bubble (air) contained in the liquid flowing in the second tube 203b.

The air bubble detection sensor 307 includes a transmitter and a receiver facing each other across a groove portion constituting a part of the guide groove 306a. In the present embodiment, the transmitter and the receiver of the air bubble detection sensor 307 face each other in the groove width direction of the groove portion. The air bubble detection sensor 307 transmits a signal from the transmitter toward the second tube 203b accommodated in the groove portion, and applies the signal to the liquid flowing in the second tube 203b from the outside of the second tube 203b made of soft vinyl chloride, for example. At this time, since a transmittance of the signal in the liquid is different from a transmittance of the signal in the bubble, even in a case where the same signals are transmitted from the transmitter, a difference occurs between the signals received by the receiver depending on whether the bubble is included in the liquid flowing through the second tube 203b. The signal transmitted from the transmitter is, for example, an ultrasonic wave, but is not limited thereto. As described above, the air bubble detection sensor 307 can detect the bubble contained in the liquid flowing in the second tube 203b on the basis of the difference between the signals received by the receiver. The air bubble detection sensor 307 is communicably connected to an acquisition unit 351 described later by wired communication or wireless communication. As a result, the air bubble detection sensor 307 can transmit, for example, information such as a signal received by the receiver to the acquisition unit 351 as bubble detection information.

The upstream-side occlusion detection sensor 308a and the downstream-side occlusion detection sensor 308b are apparatuses that detect whether or not the second tube 203b attached to the infusion pump 300 is occluded. As illustrated in Fig. 4, the upstream-side occlusion detection sensor 308a is arranged on the upstream side from the liquid delivery drive part 312 in the guide groove 306a of the tube attachment unit 306. Then, the downstream-side occlusion detection sensor 308b is arranged on the downstream side from the liquid delivery drive part 312 in the guide groove 306a of the tube attachment unit 306. In the present embodiment, the upstream-side occlusion detection sensor 308a and the downstream-side occlusion detection sensor 308b are made to have the same configuration. In the following description, in a case where the upstream-side occlusion detection sensor 308a and the downstream-side occlusion detection sensor 308b are not distinguished from each other, they are simply collectively referred to as occlusion detection sensors 308.

The occlusion detection sensor 308 includes, for example, a Hall element and a plunger that is movable with respect to the Hall element and includes a magnet. A detection surface exposed to a groove portion constituting a part of the guide groove 306a is provided at one end portion of the plunger. When the inside of the second tube 203b attached to the guide groove 306a of the tube attachment unit 306 is occluded, the diameter of the second tube 203b changes, and a pressure applied from the second tube 203b to the detection surface changes. In the occlusion detection sensor 308, the magnet moves relative to the Hall element following the pressure applied from the second tube 203b to the detection surface, so that the Hall element detects a change in magnetic flux. As a result, the occlusion detection sensor 308 can detect the pressure applied to the detection surface and detect whether or not the inside of the second tube 203b is occluded. However, the occlusion detection sensor 308 may be any apparatus capable of detecting the pressure applied to the detection surface, such as a strain gauge.

Referring again to Fig. 4, on the inner surface side of the door unit 302, a first pressing member 309a, a second pressing member 309b, and a third pressing member 309c are provided at positions facing the air bubble detection sensor 307, the upstream-side occlusion detection sensor 308a, and the downstream-side occlusion detection sensor 308b, respectively, in a state where the door unit 302 is closed with respect to the main body unit 301. When the door unit 302 is closed as illustrated in Fig. 3 after the second tube 203b is attached to the tube attachment unit 306 as illustrated in Fig. 4, the first pressing member 309a, the second pressing member 309b, and the third pressing member 309c on the door unit 302 side can press the second tube 203b against the air bubble detection sensor 307, the upstream-side occlusion detection sensor 308a, and the downstream-side occlusion detection sensor 308b, respectively. For this reason, even in a case where a plurality of types of the second tubes 203b having slight variations in outer diameter due to manufacturing tolerances or the like are attached to the infusion pump 300, the air bubble detection sensor 307, the upstream-side occlusion detection sensor 308a, and the downstream-side occlusion detection sensor 308b can more accurately detect an abnormality of the second tube 203b in a state where the door unit 302 is closed with respect to the main body unit 301. In the following description, in a case where the first pressing member 309a, the second pressing member 309b, and the third pressing member 309c are not particularly distinguished from each other, they are simply collectively referred to as pressing members 309.

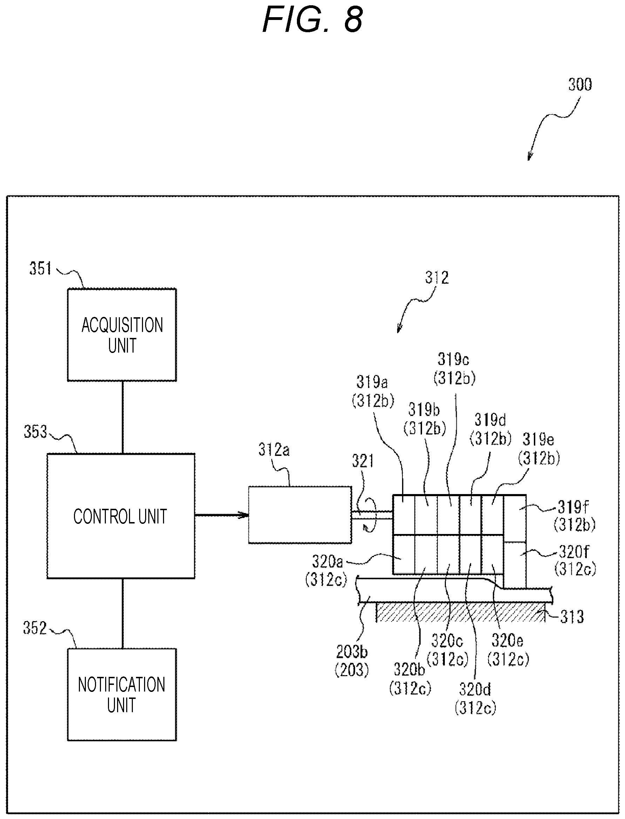

As illustrated in Fig. 8, the infusion pump 300 includes the acquisition unit 351, a notification unit 352, and a control unit 353. Fig. 8 is a block diagram of the infusion pump 300. In Fig. 8, for convenience of description, a configuration of the liquid delivery drive part 312 capable of delivering liquid in the second tube 203b is also illustrated.

The liquid delivery drive part 312 includes a drive motor 312a, a cam structure 312b including a plurality of cams rotationally driven by the drive motor 312a, and a finger structure 312c including a plurality of fingers moved by the cams of the cam structure 312b. Then, the finger structure 312c of the liquid delivery drive part 312 is arranged in the tube attachment unit 306 (see Fig. 4) described above. The cam structure 312b includes a plurality of cams, for example, six cams 319a to 319f, and the finger structure 312c includes six fingers 320a to 320f corresponding to the six cams 319a to 319f. The six cams 319a to 319f are arranged with a phase difference from each other, and the cam structure 312b is connected to an output shaft 321 of the drive motor 312a. The liquid delivery drive part 312 sequentially drives the fingers from the upstream side toward the downstream side of the second tube 203b in the extending direction of the second tube 203b. As a result, the second tube 203b is sequentially pressed and closed from the upstream side toward the downstream side of the second tube 203b, and performs peristaltic movement. For that reason, the liquid delivery drive part 312 can deliver the liquid in the second tube 203b from the upstream side toward the downstream side of the second tube 203b.

The acquisition unit 351 includes one or more communication interfaces. The acquisition unit 351 is configured to be able to acquire various types of information from other components constituting the infusion pump 300 by wired communication or wireless communication. However, the acquisition unit 351 may be configured to be able to transmit information in addition to receiving the various types of information described above. For example, the acquisition unit 351 may be able to transmit the received various types of information to an external apparatus such as a server.

For example, the acquisition unit 351 can acquire, from the occlusion detection sensors 308 (the upstream-side occlusion detection sensor 308a and the downstream-side occlusion detection sensor 308b), pressure information detected by the occlusion detection sensors 308. The pressure information detected by the occlusion detection sensor 308 is, for example, information such as an amount of change in magnetic flux detected by the Hall element of the occlusion detection sensor 308.

In addition, for example, the acquisition unit 351 can acquire the bubble detection information from the air bubble detection sensor 307. The bubble detection information is, for example, a signal itself received by a reception unit of the air bubble detection sensor 307.

In addition, for example, the acquisition unit 351 can acquire operation mode information on the infusion pump 300. Specifically, the operation panel unit 305 (see Fig. 3 and the like) of the infusion pump 300 includes a plurality of operation buttons 305a, and the acquisition unit 351 acquires the operation mode information depending on information input from the operation panel unit 305. More specifically, when the fast delivery switch button or the start switch button is pressed by an operator, the acquisition unit 351 acquires, as the operation mode information, liquid delivery mode information for executing a liquid delivery mode in which the liquid delivery drive part 312 is driven to perform liquid delivery. In addition, when the stop switch button is pressed by the operator in a state where the infusion pump 300 is in the liquid delivery mode, the acquisition unit 351 acquires, as the operation mode information, liquid delivery stop mode information for executing a liquid delivery stop mode in which the liquid delivery drive part 312 is not driven and liquid delivery is not performed.

In addition, for example, the acquisition unit 351 can acquire opened/closed state information on the door unit 302 with respect to the main body unit 301 of the infusion pump 300. Specifically, when the engaging members 314 and 315 (see Fig. 4) of the door unit 302 are fitted into the fitting portions 317 and 318 (see Fig. 4) formed in the main body unit 301, respectively, the acquisition unit 351 acquires, as the opened/closed state information, closed state information indicating that the door unit 302 is in a closed state with respect to the main body unit 301. On the other hand, when the engaging members 314 and 315 (see Fig. 4) of the door unit 302 are not in a state of being fitted into the fitting portions 317 and 318 (see Fig. 4) formed in the main body unit 301, respectively, the acquisition unit 351 acquires, as the opened/closed state information, opened state information indicating that the door unit 302 is in a state of being opened with respect to the main body unit 301.

As described above, the acquisition unit 351 can acquire information based on the operation of the infusion pump 300 itself by the operator, and can acquire information transmitted from components such as the air bubble detection sensor 307 and the occlusion detection sensor 308.

In addition, the acquisition unit 351 in the present embodiment acquires the bubble detection information from the air bubble detection sensor 307, the pressure information from the occlusion detection sensor 308, the operation mode information on the infusion pump 300, and the opened/closed state information on the door unit 302 described above, but may be made to have a configuration capable of acquiring only some of the various types of information described above, or may be made to have a configuration capable of acquiring other information in addition to the various types of information described above.

The notification unit 352 includes one or more output apparatuses. The output apparatus included in the notification unit 352 is, for example, a display, a speaker, a vibrator, or the like. The notification unit 352 outputs an image, sound, vibration, or the like.

In the present embodiment, the notification unit 352 notifies the outside of the information acquired by the acquisition unit 351 as perceptual identification information that can be identified by a human on the basis of a control command from the control unit 353 described later. In the present disclosure, the "perceptual identification information" includes any information perceivable by five human senses, such as an alarm by sound as auditory identification information, and display of a color change, turning on, turning off, blinking, or the like of light of an LED or the like as visual identification information.

The control unit 353 performs instruction on operation timing or operation for each unit of the infusion pump 300, or the like. The control unit 353 includes a processor such as a CPU or an MPU. More specifically, the control unit 353 of the present embodiment includes a read only memory (ROM), a random access memory (RAM), a non-volatile memory, and a clock. The clock can correct a current time by a predetermined operation, and can execute acquisition of the current time, measurement of an elapsed time of a predetermined liquid delivery work, measurement of a reference time of speed control of liquid delivery, and the like.

In addition, the control unit 353 is connected to a power switch button and a switch. The switch switches between a power converter unit and a rechargeable battery such as a lithium ion battery, to supply power to the control unit 353 from either the power converter unit or the rechargeable battery. The power converter unit is connected to a commercial AC power supply via an outlet.

In addition, the control unit 353 instructs a display unit driver to drive the display unit 304, and displays the scheduled amount of injection (mL) of administration described above, or various warning messages or the like on the display unit 304.

In addition, the control unit 353 controls the notification unit 352 on the basis of the various types of information acquired by the acquisition unit 351. For example, in a case where it is determined that an abnormality of the second tube 203b attached to the infusion pump 300 is detected on the basis of the information from the air bubble detection sensor 307 or the occlusion detection sensor 308 acquired by the acquisition unit 351, the control unit 353 controls the notification unit 352 to perform notification of the perceptual identification information. For example, the control unit 353 may display a warning message on the display unit 304 as the notification unit 352. Alternatively, the control unit 353 may cause a speaker as the notification unit 352 to output a warning sound. As a result, it is possible to urge a user of the infusion pump 300 to take measures, and improvement is achieved of safety of the infusion pump 300, and of usefulness of a technique for detecting an abnormality of the infusion tube 203 attached to the infusion pump 300.

In addition, the control unit 353 controls, for example, the liquid delivery drive part 312 on the basis of the various types of information acquired by the acquisition unit 351. For example, in a case where it is determined that an abnormality of the second tube 203b is detected on the basis of the information from the air bubble detection sensor 307 or the occlusion detection sensor 308 acquired by the acquisition unit 351, the control unit 353 may stop driving of the liquid delivery drive part 312 and end the liquid delivery mode in which liquid delivery is executed. As a result, improvement is achieved of safety of the infusion pump 300, and of usefulness of the technique for detecting an abnormality of the infusion tube 203 attached to the infusion pump 300.

As described above, the infusion pump 300 according to the present embodiment includes the main body unit 301 including the tube attachment unit 306 to which the infusion tube 203 (the second tube 203b in the present embodiment) is attached substantially horizontally, and the door unit 302 that is openable and closable with respect to the main body unit 301 and covers the tube attachment unit 306 in a state of being closed with respect to the main body unit 301, in which: the tube attachment unit 306 includes the indwelling portion 121 configured to place the infusion tube 203 on the upper side in the height direction of the main body unit 301, and the first protruding portion 122 that lies in a row with the indwelling portion 121, extends from the indwelling portion 121 toward the lower side in the height direction of the main body unit 301, and includes the slope 122S in which the degree of protrusion from the main body unit 301 is larger at a position closer to the lower side in the height direction of the main body unit 301; the door unit 302 includes the second protruding portion 130 protruding toward the main body unit 301 in a state where the door unit 302 is closed with respect to the main body unit 301; and the second protruding portion 130 is configured to lift the infusion tube 203 to the upper side in the height direction of the main body unit 301 along the slope 122S of the first protruding portion 122 and place the infusion tube 203 on the indwelling portion 121 when the door unit 302 is closed with respect to the main body unit 301.

With the infusion pump 300 having such a configuration, even in a case where the door unit 302 is closed with respect to the main body unit 301 in a state where the infusion tube 203 is not placed on the indwelling portion 121, the second protruding portion 130 can lift the infusion tube along the slope 122S of the first protruding portion 122 and place the infusion tube 203 on the indwelling portion 121. For this reason, it is possible to reduce a risk of occurrence of occlusion of the infusion tube 203, underdose of the liquid flowing in the infusion tube 203, or the like due to the fact that the infusion tube 203 is sandwiched between the door unit 302 and the main body unit 301 when the door unit 302 is closed with respect to the main body unit 301. Thus, with the infusion pump 300 according to the present embodiment, it is possible to improve ease of attachment of the infusion tube 203 on the infusion pump 300.

Although the present disclosure has been described with reference to the drawings and examples, it should be noted that those skilled in the art can make various modifications and corrections on the basis of the present disclosure. Thus, it should be noted that these modifications and corrections fall within the scope of the present disclosure. For example, functions and the like included in means, steps, and the like can be rearranged so as not to be logically inconsistent, and a plurality of means, steps, and the like can be combined into one, or divided.

### Industrial Applicability

The present disclosure relates to an infusion pump.

### Reference Signs List

110 Upstream-side tube guide part
111 Upper protruding portion
112 Lower protruding portion
120 Downstream-side tube guide part
121 Indwelling portion
122 First protruding portion
122S Slope
123 Upper protruding portion
130 Second protruding portion
200 Infusion line
201 Infusion container
202 Indwelling needle
203 Tube (infusion tube)
203a First tube
203b Second tube
204 Drip tube
205 Number-of-drops abnormality detection apparatus
206 Clamp
250 Stand
300 Infusion pump
301 Main body unit
302 Door unit
302a, 302b Hinge
303 Main body cover
304 Display unit
305 Operation panel unit
305a Operation button
306 Tube attachment unit
306a Guide groove
307 Air bubble detection sensor
308 Occlusion sensor
308a Upstream-side occlusion sensor
308b Downstream-side occlusion sensor
309 Pressing member
309a First pressing member
309b Second pressing member
309c Third pressing member
312 Liquid delivery drive part
312a Drive motor
312b Cam structure
312c Finger structure
313 Tube pressing member
314, 315 Engaging member
316 Lever
317, 318 Fitting portion
319a to 319f Cam
320a to 320f Finger
321 Output shaft
322 Tube clamp part
323, 324 Occlusion portion
325 Main base portion
326 Sub-base portion
327 First elastic support portion
328 Second elastic support portion
329 Distal end part
330 First locking portion
331 Second locking portion
332 Third locking portion
333 Fourth locking portion
340 Rotating arm member
341 Rotation shaft
342 Urging member
343 Pressing member
351 Acquisition unit
352 Notification unit
353 Control unit
A Liquid delivery direction
I-I' Cut surface
F1, F2, F3 External force
FIG. 3
HEIGHT DIRECTION
WIDTH DIRECTION
FIG. 4
HEIGHT DIRECTION
WIDTH DIRECTION
FIG. 5
HEIGHT DIRECTION
FIG. 7
HEIGHT DIRECTION
FIG. 8
351 ACQUISITION UNIT
352 NOTIFICATION UNIT
353 CONTROL UNIT
FIG. 9
ATTACHMENT/DETACHMENT DIRECTION
FIG. 10
HEIGHT DIRECTION
ATTACHMENT/DETACHMENT DIRECTION

## Claims

1. An infusion pump comprising:
a main body unit including a tube attachment unit to which an infusion tube is attached substantially horizontally; and
a door unit that is openable and closable with respect to the main body unit and covers the tube attachment unit in a state of being closed with respect to the main body unit, wherein
the tube attachment unit includes
an indwelling portion configured to place the infusion tube on an upper side in a height direction of the main body unit, and
a first protruding portion that lies in a row with the indwelling portion, extends from the indwelling portion toward a lower side in the height direction of the main body unit, and includes a slope in which a degree of protrusion from the main body unit is larger at a position closer to the lower side in the height direction,
the door unit includes a second protruding portion that protrudes toward the main body unit in a state where the door unit is closed with respect to the main body unit, and
the second protruding portion is configured to lift the infusion tube to the upper side in the height direction along the slope of the first protruding portion and place the infusion tube on the indwelling portion when the door unit is closed with respect to the main body unit.

2. The infusion pump according to claim 1, wherein the slope of the first protruding portion is curved in an arc shape such that the degree of protrusion from the main body unit is larger at a position closer to the lower side in the height direction.

3. The infusion pump according to claim 1 or 2, wherein the indwelling portion is provided at a position that is on a side surface in a width direction of the main body unit and is not covered by the door unit, in a state where the door unit is closed with respect to the main body unit.

4. The infusion pump according to any one of claims 1 to 3, wherein
the tube attachment unit includes a tube clamp part configured to clamp a part of the infusion tube to fix the infusion tube to the tube attachment unit, and
the first protruding portion is provided at a position where the first protruding portion is enabled to come into contact with the infusion tube in a case where the infusion tube fixed by the tube clamp part is in a state of hanging down to the lower side in the height direction by an own weight of the infusion tube.

5. The infusion pump according to claim 4, wherein the tube clamp part is configured to hold the infusion tube in an occlusion state in a state where the door unit is opened with respect to the main body unit, and hold the infusion tube in an occlusion release state in a state where the door unit is closed with respect to the main body unit.

6. The infusion pump according to claim 4 or 5, wherein the tube clamp part is detachable from the tube attachment unit.

7. The infusion pump according to any one of claims 4 to 6, wherein the second protruding portion is located on an opposite side from the tube clamp part with the first protruding portion interposed therebetween in the width direction of the main body unit in a state where the door unit is closed with respect to the main body unit.
